# EUROPEAN PATENT SPECIFICATION

(11) **EP 3 180 089 B1**
(45) Date of publication and mention of the grant of the patent: **08.07.2020**
(21) Application number: 15749938.5
(22) Date of filing: 04.08.2015
(51) Int. Cl.: A61Q 5/12, A61K 8/73

(54) **PERSONAL CARE PRODUCTS COMPRISING HIGHLY CATIONIC SUBSTITUTED STARCHES**
PRODUKTE ZUR PERSÖNLICHEN PFLEGE, DIE STARK SUBSTITUIERTE KATIONISCHE STÄRKE ENTHALTEN
PRODUITS POUR LE SOIN INDIVIDUEL, CONTENANT L'AMIDON CATIONIQUE INTENSEMENT SUBSTITUÉ

(30) Priority: 11.08.2014 US 201462036079 P; 30.07.2015 US 201514814196
(43) Date of publication of application: 21.06.2017
(73) Proprietor: Corn Products Development, Inc., Jabaquara - São Paulo, 04311-000SP (BR)
(72) Inventor: MARKLAND, Flave, Atkins, IA 52206 (US); HOSSAIN, Ashraf, Cedar Rapids, IA 52404 (US)
(74) Representative: Held, Stephan
(86) International application number: PCT/US2015/043599
(87) International publication number: WO 2016/025240

(56) References cited:
- EP-A1- 2 110 121
- GB-A- 1 285 547
- US-A1- 2009 176 675

## Description

### BACKGROUND OF INVENTION

This application claims the benefit of U.S. Provisional Application Serial No. 62/036,079 filed August 11, 2014, and U.S. Nonprovisional Application Serial No. 14/814,196, filed July 30, 2015.

The present invention is directed to personal care formulations such as shampoos, hair conditioners, body washes and the like comprising cationic starch-based components.

Conventional personal care compositions for the treatment of hair and skin typically comprise combinations of detersive surfactants and conditioning agents. Typical formulations include a detersive surfactant designed to remove oil, grease and dirt in combination with a conditioning agent such as hydrocarbon oil, fatty esters and silicone intended to condition the hair or skin. Alternatively, the compositions may omit the detersive surfactant and simply comprise the conditioning agents. Such compositions further comprise other ingredients such as fragrances, dyes, colorants, pigments, and bleaching ingredients as well as UV-absorbers, antistatic agents, preservatives and anti-microbials, anti-dandruff agents, detanglers, quaternary ammonium compounds and silicone polymer conditioning agents such as dimethicone or cyclomethicone. "Leave-on" personal care products are also known in the art which include ingredients such as moisturizers, sunscreen, lotions, combing creams, insect repellants and the like.

Of interest to the present invention are the disclosures of various patents disclosing the use of cationic polysaccharide ingredients such as cationic starch and cationic guar ingredients in personal care formulations. In particular, cationic guar gum has been found to provide desirable conditioning properties to personal care compositions.

Monin et al., US 2009/0214608 is directed to the use of cationic guar products for use in shampoos and other cosmetic compositions.

Snyder et al. US 6,248,317 discloses styling shampoo compositions comprising a surfactant and from about 0.025% to about 3% by weight of an organic cationic polymer having a molecular weight of from about 5,000 to about 10,000,000 with a charge density of from about 0.2 meq/g to about 7.0 meq/g. Cationic guar derivatives are exemplified with cationic starch said to be useful but not otherwise exemplified or disclosed.

Geary et al. US 2004/0157754 discloses an aqueous shampoo composition comprising a detersive surfactant and a cationic polymer having a molecular weight of from about 10,000 to about 10,000,000 with a charge density of from about 1.4 meq/g to about 7.0 meq/g with a conditioning agent material optionally containing silicone. Cationic guar derivatives and cationic cellulose are exemplified with cationic starch said to be useful according to the invention but not otherwise exemplified or disclosed.

Wells, US 6,930,078 discloses a shampoo composition with a detersive surfactant, a cationic guar derivative characterized by a molecular weight of from about 10,000 to about 10,000,000 and a charge density of from about 1.25 meq/g to about 7 meq/g. According to this patent the cationic guar derivatives can enhance the deposition and retention of conditioning aids and/or solid particle benefit agents on the surfaces treated therewith when incorporated in shampoo compositions.

Melby, US 6,365,140 (Calgon) is directed to the use of cationic modified starches including those from potato, corn, rice, tapioca and wheat with preferred molecular weights ranging from about 10,000 to about 10,000,000 but more preferably from 5,000 to about 500,000 and further discloses the use of cassia gum hydroxypropyltrimethyl ammonium chloride polymer as a silicone deposition aid for hair conditioners.

Johnson et al., US 8,361,448 is directed to shampoos containing gel networks comprising detersive surfactants, pre-formed solid crystalline gel network phases, and fatty acids. The shampoos are disclosed to optionally include deposition aids at levels of from about 0.05% to about 5% to enhance deposition of the gel network component which are cationic polymers selected from the group consisting of cationic cellulose derivatives, cationic starch derivatives, and cationic guar derivatives having a molecular weight from about 10,000 to about 10,000,000 and a charge density from about 0.9 meq/g to about 7.0 meq/g

Chowdhary, U.S. 2003/0129210 is directed to a flaked cationic potato with a substitution value of at least 0.01. Chowdhary teaches that conventional modified starches have been observed to have disadvantages when used in applications such as personal care and adhesives. In particular Chowdhary teaches that starch products have tended to have inferior film-forming properties as well as inferior cold water solubility, substantivity (which relates to the adherent qualities of a product such as a sunscreen and its ability to be retained after skin is exposed to water and perspiration) and lower viscosity when compared to their guar gum counterparts. Chowdhary discloses Empersol™ N branded cationic potato starch (Emsland Starch GmbH) as one suitable cationic starch. This starch is a cold water swelling wet-end potato starch with a degree of substitution (d.s.) of 0.035% which is produced over a drum drier

Erazo-Majewicz et al., US 2005/0227902 discloses the use of cationic oxidized polysaccharides having a weight average molecular weight with a lower limit of 50,000 and an upper limit of 1,000,000 and an aldehyde functionality content of at least 0.001 meq/g of polysaccharide where the polysaccharides are said to be selected from the group consisting of cellulose, starch, dextran and polygalactomannan.

Molenda US 2013/0164244 discloses hair conditioning compositions comprising oxidized starch derived cationic polymer with hydroxypropyl oxidized starch PG trimonium chloride being particularly preferred.

Dieker et al., US 8,057,786 is directed to shampoo and conditioner systems comprising cationic modified starches

Glenn, Jr. et al. US 8,273,333 is directed to a non-lathering personal care composition for application to the hair or skin comprising a non-surfactant cosmetic active, optional ionic surfactant, a plasticizer and a polymeric structurant with a weighted average molecular weight of from 40,000 to 500,000.

Also of interest to the present invention are references in which combinations of cationic polymers are mixed with various surfactants and materials. See Wells, US 8,349,300 which discloses personal care products with cationic polymers. Peffly. US 8,623,341 discloses personal care formulations such as shampoos comprising water soluble cationically modified starch polymers having molecular weights ranging from 1,000 to about 200,000 with a charge density from about 0.7 meq/g to about 7 meq/g in further combination with additional ingredients such as silicone and guar.

Peffly (US 2009/176675 A1) discloses personal care composition comprising from about 0.01 wt. % to about 10 wt. % of a water-soluble cationically modified starch polymer, which has a molecular weight from about 850,000 to about 15,000,000 and a charge density from about 0.2 meq/g to about 5 meq/g. Corresponding compositions are described as showing enhanced deposition of conditioner.

As disclosed, cationic guar has been found to be found to be particularly useful as an ingredient in personal care compositions but has some negative aesthetic impacts in use, is relatively expensive and can run in short supply. There also exists a desire to find substitutes for silicone which would provide for its reduction or elimination in compositions. Accordingly, there remains a desire in the art for personal care formulations including shampoos, conditioning shampoos and hair conditioners which perform as well as or better than prior are compositions but which have higher bio-based contents and can be silicone-free.

### BRIEF SUMMARY OF INVENTION

The cationic starches of the invention have been found to provide improved conditioning properties to cosmetic and dermatologically acceptable personal care compositions. For example, the present invention provides personal care formulations which are particularly useful in depositing silicone personal care ingredients on hair fibers much like polyquatemium ingredients and cationic guar but surprisingly provide conditioning benefits when used without any added silicone. Not only do the personal care formulations of the invention provide similar or superior functionality to commercially-available polyquatemium and cationic guar formulations they are further characterized by superior solubility profiles and higher or comparable bio-based contents than prior art products. According to a further surprising aspect of the invention it has been found that cationic starches derived from mung bean, potato, rice , waxy rice, and waxy com provide desirable conditioning properties to personal care compositions which are free of silicone polymers.

According to one aspect of the invention a personal care formulation is provided comprising: water; a second ingredient selected from the group consisting of detergents, non-detersive conditioning agents, aesthetically-modifying agents, protective agents, and mixtures thereof; and an unthinned cationic starch characterized by: a) an amylopectin/amylose weight ratio greater or equal to 60/40; b) an apparent cationic molecular weight of greater than or equal to 12 million daltons; and c) a positive charge density of from 0.5 meq/g to 2.5 meq/g. Different native starches are characterized by different molecular weights as would be appreciated by those of skill in the art so in some cases the apparent cationic molecular weight preferably ranges from 12 million to hundreds of millions daltons (for example native com starch can have a molecular weight of as much as 300 million daltons while native potato starch can have a molecular weight of as much as 500 million daltons) while in others it ranges from 12 million daltons to 35 million daltons when measured according to the methods described below. Cationic starches used according to the invention have a positive charge density of ranging from 0.5 meq/g to 2.5 meq/g or above but more preferably range from 1.0 meq/g to 2.5 meq/g and still more preferably from 1.0 meq/g to 2.0 meq/g.

Suitable cationic starches for use in the invention include selected from the group consisting of dent com, waxy com, tapioca, mung bean, potato, rice and waxy rice starch having the desired amylopectin/amylose ratios and apparent cationic molecular weights. As used herein apparent molecular weights and apparent cationic molecular weights are determined by means of a size exclusion chromatography (SEC) method which uses a combination of multiangle laser light scattering (MALLS) and refractive index (RI) detectors. According to the invention the cationic starches are characterized by an amylopectin/amylose weight ratio of greater than or equal to 60/40 and preferably from 75/25 to 92/8.

According to a further aspect of the invention cationic mung bean starch which is characterized by a cationic molecular weight of about 12 million daltons and has an amylopectin/amylose ratio similar to dent com of about 70/30.

Specifically, the invention provides a cosmetic or dermatologically acceptable composition ("rinse-off s") and methods of using those compositions to condition hair or skin, wherein the compositions comprise: water; a second ingredient selected from the group consisting of detergents and non-detersive conditioning agents as indicated above; an unthinned cationic starch characterized by: a) an amylopectin/amylose weight ratio of greater than or equal to 60/40; b) an apparent cationic molecular weight of greater than or equal to 12 million daltons; and c) a positive charge density of from 0.5 meq/g to 2.5 meq/g. The cationic starch preferably has a cationic starch has a positive charge density of from 1.0 meq/g to 2.0 meq/g. Preferred starches are selected from the group consisting of potato, rice and waxy rice starch.

According to one aspect of the invention are provided personal care compositions including, but not limited to, conditioners and conditioning shampoos wherein the cationic starches do not merely enhance the conditioning properties of components such as silicone polymers but are characterized by their own conditioning properties such that they do not require the use of additional silicone polymers. In particular, cationic starches selected from the group consisting of potato, rice, waxy rice, and waxy com starches have proven useful in such silicone-free personal care compositions.

According to one aspect of the invention the composition is a conditioning shampoo and comprises a detersive agent although alternative compositions lack a detersive agent and comprise a non-detersive conditioning agent selected from the group consisting of oily substances, non-ionic substances, cationic amphiphilic ingredients, cationic non-starch ingredients, solvents, esters, extracts, silicone polymers and polyquatemium ingredients.

Also provided are cosmetic or dermatologically-acceptable compositions ("leave-ons") comprising: water; a personal care ingredient selected from the group consisting of an aesthetically-modifying and/or protective agents; an unthinned cationic starch characterized by: a) an amylopectin/amylose weight ratio of greater than or equal to 60/40; b) an apparent cationic molecular weight of greater than or equal to 12 million daltons; and c) a positive charge density of from 0.5 meq/g to 2.5 meq/g but more preferably from 1.0 meq/g to 2.0 meq/g. Suitable aesthetically-modifying agents would be known by those of ordinary skill in the art but is preferably selected from the group comprising non-ionic surfactants, fragrances, natural oils, dyes, pigments, opacifying or pearlescent agents, hair colorants, skin colorants or nail colorants. Suitable protective agents include, but are not limited to those selected from the group comprising sunscreens, UV-absorbers, antistatic agents, preservatives, anti-microbials, and anti- dandruff agents, humectants, petrolatum, glycerin, aloe, mineral oil, ester-based emollients, antioxidants and free-radical scavengers.

Disclosed are further leave-on compositions comprising water; a personal care ingredient selected from the group consisting of an aesthetically-modifying and/or protective agents; a cationic mung bean starch characterized by: a) an apparent cationic molecular weight of greater than or equal to 10 million daltons; and c) a cationic degree of substitution of from 0.5 meq/g to 2.5 meq/g but more preferably from 1.0 meq/g to 2.0 meq/g. Suitable aesthetically-modifying agents would be known by those of ordinary skill in the art but is preferably selected from the group comprising non-ionic surfactants, fragrances, natural oils, dyes, pigments, opacifying or pearlescent agents, hair colorants, skin colorants or nail colorants. Suitable protective agents include, but are not limited to those selected from the group comprising sunscreens, UV- absorbers, antistatic agents, preservatives, anti-microbials, and anti-dandruff agents, humectants, petrolatum, glycerin, aloe, mineral oil, ester-based emollients, antioxidants and free-radical scavengers.

Those of ordinary skill in the art would be able to determine the appropriate concentration of unthinned cationic starch depending on the intended use of the composition but according to one use the cationic starch is present in at a concentration of from 0.1% to 3% w/w with concentrations of from 0.5% to 2% w/w being more preferred.

According to a further aspect of the invention it has been discovered that cationic mung bean starch is particularly useful as an ingredient in cosmetic or dermatologically acceptable personal care compositions and methods of using those compositions to condition hair or skin. Specifically, a cosmetic or dermatologically acceptable composition is diclosed comprising: water; a personal care ingredient selected from the group consisting of detergents and non-detersive conditioning agents; a cationic mung bean starch characterized by: a) an apparent cationic molecular weight of from 10 million to 35 million daltons or greater up to its native molecular weight; and b) a cationic degree of substitution of from 0.5 meq/g to 2.5 meq/g. Conventional detergents may be incorporated into the compositions as would be appreciated by those of skill in the art. Alternatively, non-detersive conditioning agents selected from the group consisting of oily substances, non-ionic substances, cationic amphiphilic ingredients, cationic non-starch ingredients, solvents, esters, extracts, silicone polymers and polyquatemium ingredients.

### BRIEF DESCRIPTION OF THE DRAWINGS

Fig. 1 depicts a box plot of the maximum wet combing load of shampoos with various ingredients without silicone;
Fig. 2 depicts a box plot of the maximum wet combing load of shampoos with various ingredients with silicone;
Fig. 3 depicts a box plot of the maximum wet combing load of sulfate free shampoos;
Fig. 4 depicts a box plot of the maximum wet combing load of shampoos without silicone at a reduced test polymer concentration; and
Fig. 5 depicts a box plot of the maximum wet combing load of shampoos without silicone at a reduced test polymer concentration

### DETAILED DESCRIPTION OF INVENTION

The present invention provides personal care formulations which are particularly useful in depositing silicone personal care ingredients on hair fibers much like polyquaternium ingredients and cationic guar. The cationic starches of the invention have been found to provide improved conditioning properties to cosmetic and dermatologically acceptable personal care compositions. Of particular interest to the present invention is the fact that the cationic starches being used are generally unthinned and are used at molecular weights close to those of their native starches. This is in contrast to prior art formulations which generally use thinned and sometimes significantly thinned starches. Not only do the personal care formulations of the invention provide similar or superior functionality to commercially-available polyquaternium and cationic guar formulations they are further characterized by superior solubility profiles and higher or comparable bio-based contents than prior art products.

According to one aspect of the invention the unthinned cationic starch ingredients derived from potato, rice, waxy rice, and waxy corn not only enhance the deposition of silicone conditioning polymers but surprisingly provide conditioning benefits when used without any added silicone. Not only do the personal care formulations of the invention provide similar or superior functionality to commercially available polyquaternium and cationic guar formulations they are further characterized by superior solubility profiles and comparable or higher bio-based contents than prior art products. According to a further surprising aspect of the invention it has been found that cationic starches derived from mung bean, potato, rice and starches provide desirable conditioning properties to personal care compositions which are free of silicone polymers.

According to one aspect of the invention a personal care formulation is provided comprising: water; a second ingredient as indicated in claim 1 and an unthinned cationic starch characterized by: a) an amylopectin/amylose weight ratio of greater than or equal to 60/40; b) an apparent cationic molecular weight of greater than or equal to 12 million daltons; and c) a positive charge density of from 0.5 meq/g to 2.5 meq/g. Suitable unthinned cationic starches for use in the invention include selected those from the group consisting of potato, rice, waxy rice, and waxy corn having the desired amylopectin/amylose ratios and apparent cationic molecular weights.

Further disclosed is cationic mung bean starch which is characterized by a cationic molecular weight of about 10 million daltons but has an amylopectin/amylose ratio of close to 60/40, which is also surprising useful in personal care formulations and can be used not only to enhance the deposition of silicone polymers and improve their conditioning effects but also to replace silicone polymers and provide silicone-free personal care compositions with improved conditioning properties

According to one aspect of the invention a particularly useful dry, unthinned cationic potato starch for personal care conditioning applications is produced which falls under CAS#56780-58-6. The starch has a positive charge density that can vary from 0.7 and 2.5 meq/g. It is an alternative to naturally derived Polyquatemium-10, CAS# 81859-24-7 and Cationic guar, CAS# 65497-29-2 as well as synthetic cationic polymers such as Polyquatemium-6 (PQ-6) and Polyquatemium-7 (PQ-7) and the like.

The personal care compositions of the invention include those of the "rinse off' category such as shampoos, rinse-off conditioners, body washes, facial washes, liquid and bar soaps, hydro-alcoholic based products such as hand sanitizers and the like which can be readily washed off with water and those categorized as "leave-ons" such as sunscreen, lotions, combing creams, insect repellants and the like which are intended to remain on the skin or hair for an extended period. Additional leave-ons include color cosmetics such as pigmented skin colorants, nail polish and nail polish remover, mascara, rouge, lipstick and balm.

The unthinned cationic starch components of the invention are combined with cosmetic or dermatological ingredients in cosmetically or dermatologically acceptable media which are compatible with application to keratinous substances such as skin and hair. The compositions preferably have a pH of from about 1 to about 13 and more preferably from 2 to 12 but those of ordinary skill in the art would understand to select a pH most compatible with the body part to which the compositions are being applied and the components within the personal care ingredients. Organic and inorganic acids may be used to adjust the pH and provide other properties to the compositions as would be appreciated by those of ordinary skill.

The cosmetically or dermatologically acceptable medium can comprise water and/or other organic solvents including hydrophilic, lipophilic and amphiphilic solvents generally known to the art. Various branched and unbranched fatty acids and fatty alcohols are also useful in the cosmetic and dermatological compositions of the invention as would be appreciated by those of ordinary skill in the art. Other lipophilic compounds including waxes of animal, vegetable or mineral origin may also be incorporated into the compositions of the invention.

The compositions can also comprise carbohydrate compounds including gums, sugars and other modified and unmodified monosaccharides, oligosaccharides and polysaccharides. Particularly suitable compounds are those described by Dubief et al., US 7,211,268, the disclosure of which is hereby incorporated by reference in its entirety and which describes such compounds as including glucans; modified or unmodified starches, such as those from cereals, for example wheat, maize and rice, from vegetables such as white peas, from tubers such as potatoes and cassava; or palm tree pith such as sago starch, amylose, amylopectin, glycogen, dextrans; celluloses and their derivatives such as methyl celluloses, hydroxyalkyl celluloses, ethylhydroxyethyl celluloses, carboxymethyl celluloses, fructosans, inulin, levan, mannans, xylans, lignins, arabans, galactans, galacturonans; chitin, chitosans and derivatives thereof; glucoronoxylans, arabinoxylans, xyloglucans; glucomannans; pectic acids and pectins; alginic acid and alginates; arabinogalactans, carrageenans, agars, glycosaminoglucans, gum arabics, tragacanth gums, ghatti gums, karaya gums, carob gums, xanthan gums, cyclodextrins, and mixtures thereof.

The compositions can further comprise antioxidants and free radical scavengers known to the art such as Vitamin E (tocopherol) and Vitamin C (ascorbic acid). They can also include chelating agents such as ethylenediaminetetraacetic acid (EDTA) and the like.

Ingredients such as UV-absorbers (such as bemotrizinol, avobenzone, padimate 0, octinosate, oxybenzone, sulisobenzone, octisalate, octocrylene), antibacterial and antifungal ingredients and other therapeutic ingredients such as anti-dandruff agents (e.g., zinc pyrithione, coal tar) can also be incorporated into the compositions of the invention.

The unthinned cationic starches of the invention may be incorporated in personal care products at concentrations which could readily be determined by those of skill in the art but are preferably incorporated as concentrations below 1.5%, more preferably below 0.7% and most preferably below 0.5%.

Anionic surfactants useful in the personal care compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, and combinations thereof.

Still other suitable anionic surfactants are the reaction products of fatty acids esterified with isethionic acid and neutralized with sodium hydroxide where, for example, the fatty acids are derived from coconut oil or palm kernel oil, and sodium or potassium salts of fatty acid amides of methyl tauride where, for example, the fatty acids are derived from coconut oil or palm kernel oil.

Other anionic surfactants suitable for use in the shampoo compositions are the succinnates, examples of which include disodium N-octadecylsulfosuccinnate, disodium lauryl sulfosuccinate, diammonium lauryl sulfosuccinate, tetrasodium N-(1,2-dicarboxyethyl)-N-octadecylsulfosuccinnate, diamyl ester of sodium sulfosuccinic acid, dihexyl ester of sodium sulfosuccinic acid, and dioctyl esters of sodium sulfosuccinic acid.

Other suitable anionic surfactants include olefin sulfonates having about 10 to about 24 carbon atoms. In this context, the term "olefin sulfonates" refers to compounds which can be produced by the sulfonation of alpha-olefins by means of uncomplexed sulfur trioxide, followed by neutralization of the acid reaction mixture in conditions such that any sulfones which have been formed in the reaction are hydrolyzed to give the corresponding hydroxy-alkanesulfonates. The alpha-olefins from which the olefin sulfonates are derived are mono-olefins having from about 10 to about 24 carbon atoms, preferably from about 12 to about 16 carbon atoms. Preferably, they are straight chain olefins.

Another class of anionic surfactants suitable for use herein is the beta-alkyloxy alkane sulfonates. These surfactants conform to the formula: 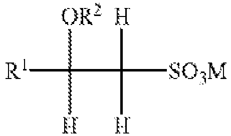 where R¹ is a straight chain alkyl group having from about 6 to about 20 carbon atoms, R² is a lower alkyl group having from about 1 to about 3 carbon atoms, preferably 1 carbon atom, and M is a water-soluble cation as described.

In addition to the sulfates, isethionates, sulfonates, sulfosuccinates described above, other potential anions for the anionic surfactant include phosphonates, phosphates, and carboxylates.

The personal care compositions of the present invention may also include one or more additional surfactants selected from the group consisting of amphoteric surfactants, zwitterionic surfactants, cationic surfactants, and nonionic surfactants. Suitable amphoteric, zwitterionic, cationic, or nonionic surfactants for use in the personal care compositions herein include those which are known for use in hair care or other personal care compositions. The concentration of such surfactants preferably ranges from about 0.5% to about 20%, preferably from about 1% to about 10%, by weight of the composition. Non-limiting examples of suitable surfactants are described in U.S. Pat. Nos. 5,104,646 and 5,106,609, both to Bolich, Jr. et al. Non-limiting examples of other surfactants suitable for use in the personal care compositions are described in McCutcheon's, Emulsifiers and Detergents, 1989 Annual, published by M. C. Publishing Co.

The surfactants are preferably included in the formulations at concentrations below 35%, more preferably 20% and most preferably below 15%. Other optional ingredients include compounds and mixtures that modify the aesthetics of the final composition. Fragrances and natural oils may be used to provide a desirable odor while dyes, pigments, opacifying or pearlescent agents can be used to impart a more appealing appearance. From a performance perspective, silicones can be employed to impart a conditioning benefit such as reduced wet combing force or enhanced shine, while Vitamins, amino acids and humectants can provide enhanced protective and reparative functionality. Of particular utility are ingredients designed to protect, prolong or enhance the intensity of hair colorants or skin and nail coloring compositions.

Non-detersive conditioning agents useful in practice of the invention are well known in the art and may be selected from among a group of well-known categories. Particularly useful conditioning agents can be selected from among oily substances, non-ionic substances, cationic amphiphilic ingredients, cationic polymers and mixtures thereof. Oily substances are selected from such as natural oils such as olive oil, almond oil, avocado oil, wheat germ oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Moisturizing agents such as panthenols and polyols, such as glycerol, polyethylene glycols with molecular weight 200 to 20,000 can also be present as non-detersive conditioning agents. The moisturizing ingredients can be included in the conditioner compositions at a concentration range of 0.01-2.5% by weight calculated to the total composition. Additional moisturizing agents include ester-based emollients such as cetyl lactate, lauryl lactate, C-12 to C-15 lactate, dicetyl malate, myristyl lactate, decyl oleate, isodecyl oleate, diisopropyl adipate, isocetyl alcohol, isodecyl neopentanoate, ethylhexyl palmitate, isocetyl stearate, myristyl myristate and myristyl laurate, glycidyl dilaurate, tridecyl neopentanoate, isostearyl neopentanoate, octyldodecyl stearate, isocetyl stearoyl stearate, octyldodecyl stearoyl stearate, carpylic/capric triglyceride.

Oily substances may also be selected from commercially available silicones such as dimethicones, dimethiconols, polydimethylsiloxanes, arylated silicones, cyclic silicones, silicone surfactants, aminated silicones and arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane.

Commercially available silicones include cyclomethicone, cycloheptasiloxane, cyclohexasiloxane, cyclopentasiloxane, cyclotetrasiloxane, and cyclotrisiloxane and further include silicone surfactants and aminated silicone surfactants such as silicone quaternium compounds and the like.

Further, any of a variety of polyquaternium compounds are also useful as non-detersive conditioning agents according to the invention.

The non-ionic conditioning agents may be incorporated in the range of 0.01 to 10%, preferably 0.05 to 7.5%, more preferably 0.1 to 5% and most preferably 0.1 to 3% by weight calculated to total composition.

Non-starch cationic ingredients may also be used as non-detersive conditioning agents such as cetyltrimethyl ammonium chloride, steartrimonium chloride, behentrimonium chloride, stearamidopropyl trimonuim chloride, dioleoylethyl dimethyl ammonium methosulfate, and dioleoylethyl hydroxyethylmonium methosulfate. Amido amines such as stearamidopropyl dimethyl amine may also be used as well as a conditioning cationic surfactant in the compositions of the present invention.

Other suitable conditioning ingredients include glyceryl ethers such as glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether, glyceryl lauryl ether, glyceryl myristyl ether, glyceryl palmityl ether, glyceryl stearyl ether and glyceryl behenyl ether and their mixtures. Most preferred are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether are glyceryl lauryl ether, and the like.

Still other non-detersive conditioning ingredients include polyphenols such as those derived in aqueous and alcoholic plant extracts. Suitable extracts include those derived from aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn and the like.

The personal care formulations of the invention may also contain other ingredients to improve their appearance and consumer appeal such as fragrances, dyes, colorants, pigments, bleaches, pearlescent agents such as mica, titanium dioxide coated mica, opacifying agents and the like. Rheology modifiers such as carbomer, poly(vinylpyrrolidone), hydroxypropyl methylcellulose, hydroxypropyl cellulose, sodium polyacrylate can be employed to provide thickening or other aesthetically-pleasing characteristics.

### EXAMPLES

The conditioning properties of a standard shampoo composition containing various cationic substituted starches were evaluated. All testing was performed on hair procured from International Hair Importers & Products (Glendale, NY). The hair tresses weighed approximately 3 g and measured 20.3 cm (8") in length and 2.54 cm (1") in width. Prior to testing, the tresses were bleached using a 6% hydrogen peroxide at a pH of 10.2. The tresses were left in contact with the bleach solution for 40 minutes under controlled temperature conditions (40°C). At the end of this process, tresses were thoroughly rinsed under an Intellifaucet rinsing apparatus set at 40°C with a controlled flow rate of 1.0 GPM. Various shampoo formulations with and without Silicone were produced and tested.

### Shampoo formulations

Shampoos with Silicone:

| | |
|---|---|
| Surfactant blend (Miracare® LSC-217 ¹) | 25% |
| Silicone (Dow 200 350Ccst) | 1.5% |
| Test polymer | 1% |
| Water | qsp 100 |

Shampoos without Silicone:

| | |
|---|---|
| Surfactant blend (Miracare® LSC-217 ¹) | 25% |
| Test polymer | 1% |
| Water | qsp 100 |

| | |
|---|---|
| ¹(Sodium Lameth Sulfate, Water, Cocamide MEA, Cocamidopropyl Betaine with 64-67% actives) | |

### Alternative Sulfate-free Shampoo formulations

Shampoos with Silicone:

| | |
|---|---|
| Surfactant blend (Miracare® Plaisant²) | 44% |
| Silicone (Dow 200 350Ccst) | 1.5% |
| Test polymer | 1% |
| Water | qsp 100 |

Shampoos without Silicone:

| | |
|---|---|
| Surfactant blend (Miracare® Plaisant²) | 44% |
| Test polymer | 1% |
| Water | qsp 100 |

| | |
|---|---|
| ²INCi Listing: (Water, Sodium Cocoyl Isethionate, Sodium Lauroamphoacetate, Sodium Methyl Cocoyl Taurate with 35-38% actives and a preservative: Neolone® 950) | |

All product treatments were conducted according to the following procedure: Shampoo was applied at a quantity of 0.1 g/g of hair of shampoo applied to tress of wet hair and massaged in between thumb and forefinger for thirty (30) seconds. The tress is then rinsed under Intellifaucet rinsing apparatus set at 38°C and 3.78 1/min (1 gal/min) for 30 seconds.

### Wet Combing Force

The primary technical function of most conditioning products is to lubricate the hair surface; and, in doing so, facilitate manageability and provide detangling benefits and lower combing friction. A common and highly consumer-relevant approach for measuring this lubrication involves an instrumental combing experiment. Testing involves use of an Instron brand tensile tester to measure frictional forces while a hair tress is pulled through a comb. Wet combing force was measured by testing eight (8) tresses (each 3.0g, 20.3 cm (8inch) in length) were used per treatment group. The tests are carried out in accordance with the method first proposed by Garcia & Diaz (JSCC, 27, (1976) 379-398 - Combability Measurements on Hair). Combing experiments were performed in the wet state after treatment. Six (6) measurements were taken per swatch using an Instron brand tensile tester to evaluate product performance and are reported in units of Grams of Force (gF) where 1 gF equals about 0.0098 Newtons.

### Shine

The commercially-available Samba device by Bossa Nova was used to quantify shine on hair tresses. The equipment operates in accordance with a collection of referenced literature articles (e.g. Bustard & Smith, Appl.Optics, 30, (1991), 3485; McMullen and Jachowicz, JSCC, 54, (2003), 335; McMullen and Jachowicz, JSCC, 55, (2004), 29) whereby the ratio of polarized and non-polarized light reaching the detector as an indicator of specular and diffuse reflection. These two values can then be employed in accordance with any of the equations in the scientific literature to produce numerical shine values.

The intensity, breadth and contrast of the shine bands on the hair affect the impression of shine and are quantified as shine indices which are calculated from the measurement in the Samba device. These phenomena may be affected by hair color, reflectivity of the fibers' surface as well as the degree of alignment of the hair fibers. A commercial tress holder that does not impose a manual alignment of the hair fibers was used to allow this to be a driving variable between samples. Consequently, devices that provide a higher degree of alignment may yield a higher shine index. In all instances, four shine measurements were performed on each tress, with eight replicate tresses being used per sample to ensure statistical relevance. All experiments were performed on bleached hair. The shine data show that use of the ingredients of the invention does not adversely affect shine.

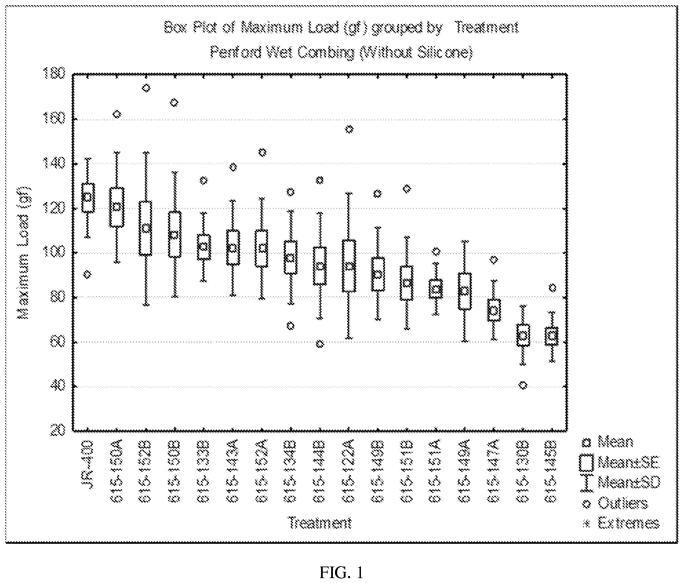

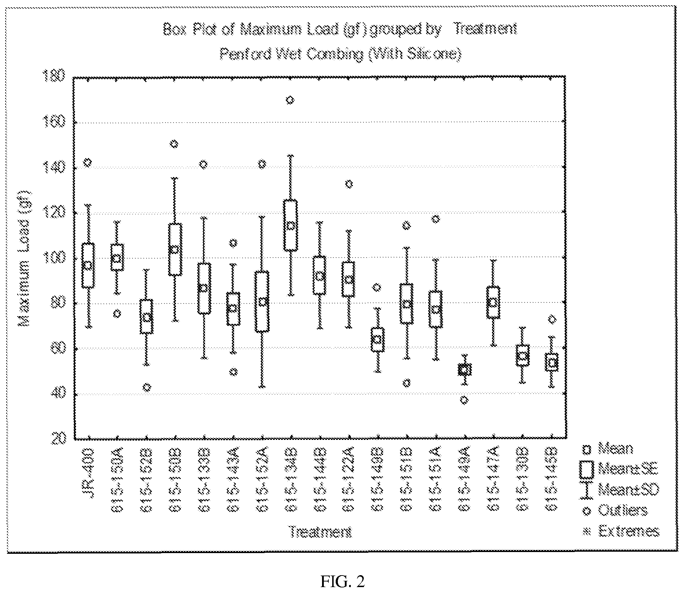

The wet combing results of conventional shampoo formulations including sulfates (the Miracare® LSC-217 shampoo base) containing cationic starches are show in Tables 1A, 1B, 2 and 3 below and in Figs. 1 and 2. The results in Figs. 1 and 2 are shown using box & whisker plots using Statistica™ while JMP™ analytical software was used to perform the statistical analysis. Statistics were performed using the student's t-test at the 95% confidence level.

**Table 1A**

| Batch ID | Base Material | Mutek meq/g | Cationic Apparent MW x 10⁶ | Amylopectin/ Amylose | Mean Combing Force gF (w/out Silicone) | Mean Combing Force gF (w/ Silicone) |
|---|---|---|---|---|---|---|
| JR-400 | PQ-10 HEC | 1.48 | 0.6 | na | 124.8 | 96.7 |
| 615-150A | Low MW Potato | 1.57 | 1.1 | 73/27 | 120.6 | 100.3 |
| 615-152B | Pea | 1.34 | 20.9 | 56/44 | 111.0 | 74.2 |
| 615-150B | High MW Potato | 1.22 | 7.8 | 82/18 | 108.4 | 103.8 |
| 615-133B | Potato | 0.83 | 21.2 | 84/16 | 102.7 | 86.7 |
| 615-143A | Dent Corn | 1.33 | 17.7 | 70/30 | 102.3 | 77.6 |
| 615-152A | Tapioca | 1.32 | 17.5 | 67/33 | 102.1 | 80.6 |
| 615-134B | Potato | 0.57 | 76.7 | 81/19 | 98.0 | 114.1 |
| 615-144B | 70% Amylose | 1.48 | 5.4 | 30/70 | 94.3 | 91.0 |
| 615-122A | Potato | 1.10 | 11.1 | 84/16 | 94.2 | 90.6 |
| 615-149B | Waxy Dent | 1.28 | 23.8 | 91/9 | 90.7 | 63.5 |
| 615-151B | Waxy Rice | 1.33 | 27.0 | 90/10 | 86.6 | 79.6 |
| 615-151A | Rice | 1.31 | 19.1 | 80/20 | 83.8 | 77.0 |
| 615-149A | Potato | 1.33 | 21.8 | 84/16 | 82.9 | 50.4 |
| 615-147A | Mung Bean | 1.39 | 15.6 | 70/30 | 74.4 | 79.9 |
| 615-130B | Guar Gum | 0.66 | 1.2 | na | 63.2 | 56.7 |
| 615-145B | Potato | 1.99 | 15.6 | 84/16 | 62.6 | 53.6 |

**Table 1B**

| Batch ID | Base Material | Mutek meq/g | Cationic Apparent MW X 10⁶ | Amylopectin/ Amylose | Mean Combing Force gF (w/out Silicone) | Mean Combing Force gF (w/ Silicone) |
|---|---|---|---|---|---|---|
| 615-134B | Potato | 0.57 | 76.7 | 81/19 | 98 | 114.1 |
| 615-150B | High MW Potato | 1.22 | 7.8 | 82/18 | 108.4 | 103.8 |
| 615-150A | Low MW Potato | 1.57 | 1.1 | 73/27 | 120.6 | 100.3 |
| JR-400 | PQ-10 HEC | 1.48 | 0.6 | Na | 124.8 | 96.7 |
| 615-144B | 70% Amylose | 1.48 | 5.4 | 30/70 | 94.3 | 91 |
| 615-122A | Potato | 1.1 | 11.1 | 84/16 | 94.2 | 90.6 |
| 615-133B | Potato | 0.83 | 21.2 | 84/16 | 102.7 | 86.7 |
| 615-152A | Tapioca | 1.32 | 17.5 | 67/33 | 102.1 | 80.6 |
| 615-147A | Mung Bean | 1.39 | 15.6 | 70/30 | 74.4 | 79.9 |
| 615-151B | Waxy Rice | 1.33 | 27 | 90/10 | 86.6 | 79.6 |
| 615-143A | Dent Corn | 1.33 | 17.7 | 70/30 | 102.3 | 77.6 |
| 615-151A | Rice | 1.31 | 19.1 | 80/20 | 83.8 | 77 |
| 615-152B | Pea | 1.34 | 20.9 | 56/44 | 111 | 74.2 |
| 615-149B | Waxy Den | 1.28 | 23.8 | 91/9 | 90.7 | 63.5 |
| 615-130B | Guar Gum | 0.66 | 1.2 | na | 63.2 | 56.7 |
| 615-145B | Potato | 1.99 | 15.6 | 84/16 | 62.6 | 53.6 |
| 615-149A | Potato | 1.33 | 21.8 | 84/16 | 82.9 | 50.4 |

**Table 2**

| Results from Wet Combing without Silicone | | | | | | | | | | |
|---|---|---|---|---|---|---|---|---|---|---|
| Treatment | N | Mean gF | Std Dev | Std Err Mean | | | | | | |
| JR-400 | 8 | 124.83 | 17.67 | 6.25 | A | | | | | |
| 615-150A | 8 | 120.58 | 24.69 | 8.73 | A | B | | | | |
| 615-152B | 8 | 111.02 | 34.12 | 12.07 | A | B | C | | | |
| 615-150B | 8 | 108.37 | 28.00 | 9.90 | A | B | C | | | |
| 615-133B | 8 | 102.69 | 15.35 | 5.43 | | B | C | D | | |
| 615-143A | 8 | 102.33 | 21.24 | 7.51 | | B | C | D | | |
| 615-152A | 8 | 102.06 | 22.45 | 7.94 | | B | C | D | | |
| 615-134B | 8 | 98.04 | 20.69 | 7.31 | | | C | D | | |
| 615-144B | 8 | 94.26 | 23.44 | 8.29 | | | C | D | E | |
| 615-122A | 8 | 94.23 | 32.48 | 11.49 | | | C | D | E | |
| 615-149B | 8 | 90.70 | 20.56 | 7.27 | | | C | D | E | |
| 615-151B | 8 | 86.55 | 20.75 | 7.33 | | | | D | E | |
| 615-151A | 8 | 83.84 | 11.39 | 4.03 | | | | D | E | F |
| 615-149A | 8 | 82.91 | 22.41 | 7.93 | | | | D | E | F |
| 615-147A | 8 | 74.39 | 13.18 | 4.66 | | | | | E | F |
| 615-130B | 8 | 63.20 | 13.24 | 4.68 | | | | | | F |
| 615-145B | 8 | 62.63 | 11.02 | 3.90 | | | | | | F |

Levels not connected by same letter are significantly different.

Treatments 615-130B and 615-145B gave rise to substantially lower combing forces when compared with JR-400. Treatment 615-145B has the lowest wet combing force and shows a decrease in wet combing force of approximately 50% when compared to treatment JR-400 only.

**Table 3**

| Results from Wet Combing with Silicone | | | | | | | | | | | | |
|---|---|---|---|---|---|---|---|---|---|---|---|---|
| Treatment | N | Mean gF | Std Dev | Std Err Mean | | | | | | | | |
| 615-134B | 8 | 114.14 | 30.88 | 10.92 | A | | | | | | | |
| 615-150B | 8 | 103.80 | 31.60 | 11.17 | A | B | | | | | | |
| 615-150A | 8 | 100.35 | 15.84 | 5.60 | A | B | C | | | | | |
| JR-400 | 8 | 96.71 | 26.85 | 9.50 | A | B | C | D | | | | |
| 615-144B | 8 | 92.00 | 23.35 | 8.26 | A | B | C | D | | | | |
| 615-122A | 8 | 90.64 | 21.28 | 7.52 | | B | C | D | | | | |
| 615-133B | 8 | 86.73 | 30.94 | 10.94 | | B | C | D | | | | |
| 615-152A | 8 | 80.61 | 37.70 | 13.33 | | | C | D | E | | | |
| 615-147A | 8 | 79.88 | 18.80 | 6.65 | | | C | D | E | | | |
| 615-151B | 8 | 79.59 | 24.29 | 8.59 | | | C | D | E | | | |
| 615-143A | 8 | 77.61 | 19.49 | 6.89 | | | C | D | E | F | | |
| 615-151A | 8 | 77.01 | 21.88 | 7.74 | | | | D | E | F | | |
| 615-152B | 8 | 74.16 | 20.92 | 7.40 | | | | D | E | F | G | |
| 615-149B | 8 | 63.53 | 14.01 | 4.95 | | | | | E | F | G | H |
| 615-130B | 8 | 56.67 | 12.16 | 4.30 | | | | | | F | G | H |
| 615-145B | 8 | 53.59 | 10.84 | 3.83 | | | | | | | G | H |
| 615-149A | 8 | 50.36 | 6.61 | 2.34 | | | | | | | | H |

The shampoo comprising 615-149A cationic potato starch gave rise to substantially lower combing forces when compared with Polyquaternium-10 (Dow U-Care™ Polymer JR-400) and has the lowest wet combing force and shows a decrease in wet combing force of approximately 48% when compared to treatment Polyquaternium-10 only.

Shampoo compositions with and without silicone comprising cationic potato starch with medium (1.33 meq/g) and high (1.99 meq/g) charges and cationic mung bean starch with a medium cationic charge (1.39 meq/g) according to the invention were tested against shampoos made with Polyquaternium-10 (Dow U-Care™ Polymer JR-400) and with cationic guar gum (Ashland N-Hance™ 3196) for mean combing force with the results shown in Table 4 below.

**Table 4**

| Batch ID | Sample ID | Mutek meq/g | Apparent Cationic MW x 10⁶ | Amylopectin/ Amylose | Mean Combing Force gF (w/out Silicone) | Mean Combing Force gF (w/ Silicone) |
|---|---|---|---|---|---|---|
| JR-400 | PQ-10 (Control) | 1.48 | 0.6 | na | 124.8 | 96.7 |
| 615-130B | N-Hance 3196 Cationic Guar Gum | 0.66 | 1.2 | na | 63.2 | 56.7 |
| 615-149B | Waxy Dent Starch | 1.28 | 23.8 | 91/9 | 90.7 | 63.53 |
| 615-149A | Potato Starch | 1.33 | 21.8 | 84/16 | 82.9 | 50.4 |
| 615-145B | Potato Starch | 1.99 | 15.6 | 84/16 | 62.6 | 53.6 |
| 615-147A | Mung Bean Starch | 1.39 | 15.6 | 70/30 | 74.4 | 79.9 |

The results show that the formulations of the invention provide combing qualities superior to compositions comprising polyquaternium-10 and roughly equivalent to those provided by cationic guar gum.

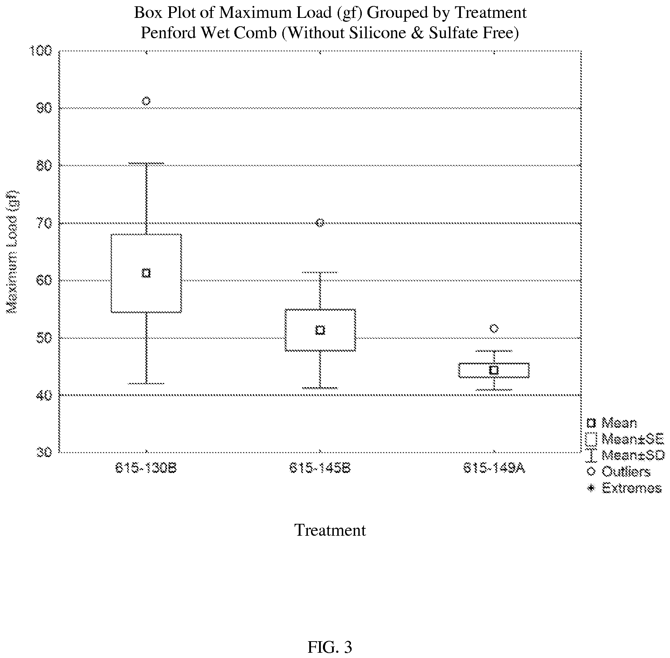

Sulfate-free shampoo formulations using the Miracare® Plaisant formulation described above were also tested according to the above-described protocol with sample 615-130B comprising cationic guar gum, 615-145B comprising highly charged cationic potato starch and sample 615-149A comprising medium charged potato starch. The results shown in Fig. 3 demonstrate a trend of lowered combing forces for the cationic potato and waxy corn compared with cationic guar gum containing formulations.

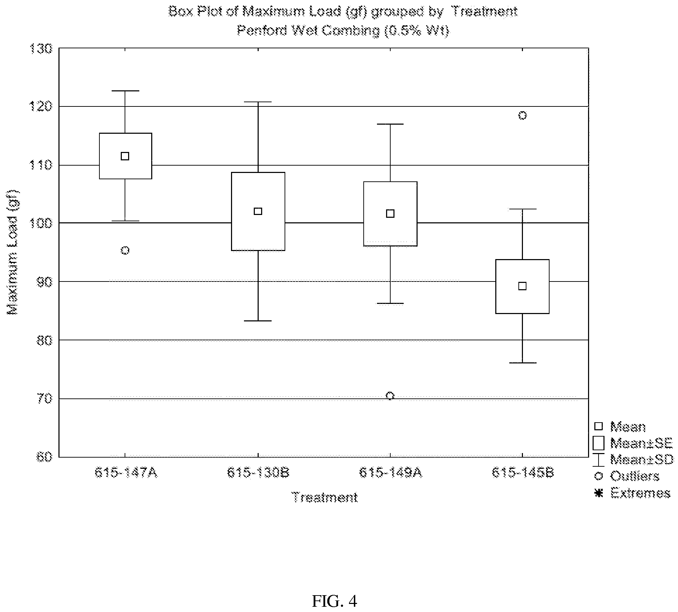

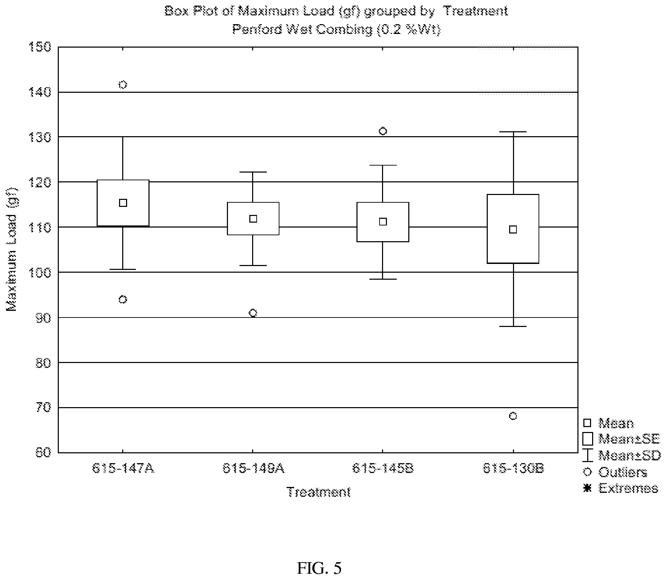

Shampoo formulations using the Miracare® LSC-217 formulation described above were tested in accordance with the methods described above with samples 615-130B comprising cationic guar gum, 615-145B comprising highly charged cationic potato starch, 615-147A comprising cationic mung bean starch and 615-149A comprising medium charge waxy dent starch but comprising reduced levels of the test polymer (0.5% wt.) and (0.2% wt.) with the results shown in Figs. 4 and 5. Those results show that the compositions of the invention provide wet combing results roughly equal to those of the cationic guar control.

### Shampoos without Silicone:

| | |
|---|---|
| Surfactant blend (Miracare® LSC-217¹) | 25% |
| Test polymer | (0.5%) or (0.2%) |
| Water | qsp 100 |

| | |
|---|---|
| ¹(Sodium Laureth Sulfate, Water, Cocamide MEA, Cocamidopropyl Betaine with 64-67% actives) | |

The invention provides a wide variety of cosmetic and dermatologically acceptable personal care formulations as illustrated below. Those of ordinary skill in the art would appreciate that these formulations may be modified and that other personal care formulations can be produced including the cationic starches described herein.

### Shampoo with Silicone

**Table 5**

| **Ingredient** | **Common Name** | **%** | **%** | **%** | **%** | **%** |
|---|---|---|---|---|---|---|
| DI Water | Water | 74.38 | 74.38 | 74.38 | 74.38 | 74.38 |
| U Care Polymer JR-400 | Polyquaternium 10 | 0.50 | | | | |
| N-Hace 3196 | Guar Hydroxypropyltrimonium Chloride | | 0.50 | | | |
| 615-149A | Cationic Potato Starch | | | 0.50 | | |
| 615-145B | Cationic Potato Starch | | | | 0.50 | |
| 615-147A | Cationic Mung Bean Starch | | | | | 0.50 |
| Texapon N 70 NA | Sodium Laureth Sulfate | 12.85 | 12.85 | 12.85 | 12.85 | 12.85 |
| Amphosol CG | Cocamidopropyl Betaine | 8.57 | 8.57 | 8.57 | 8.57 | 8.57 |
| Sodium Chloride USP | Sodium Chloride | 1.00 | 1.00 | 1.00 | 1.00 | 1.00 |
| Fragrance | Fragrance | 0.60 | 0.60 | 0.60 | 0.60 | 0.60 |
| Edeta BD | Disodium EDTA | 0.10 | 0.10 | 0.10 | 0.10 | 0.10 |
| Xiameter MEM-1785 Emulsion | Dimethiconol, TEA-Dodecylbenzenesulfonate | 1.00 | 1.00 | 1.00 | 1.00 | 1.00 |
| Preservatives, pH Adjuster | | 1.00 | 1.00 | 1.00 | 1.00 | 1.00 |
| | | 100 | 100 | 100 | 100 | 100 |

### Shampoo No Silicone

**Table 6**

| **Ingredient** | **Common Name** | **%** | **%** | **%** | **%** | **%** |
|---|---|---|---|---|---|---|
| DI Water | Water | 75.38 | 75.38 | 75.38 | 75.38 | 75.38 |
| U Care Polymer JR-400 | Polyquaternium 10 | 0.50 | | | | |
| N-Hace 3196 | Guar Hydroxypropyltrimonium Chloride | | 0.50 | | | |
| 615-149A | Cationic Potato Starch | | | 0.50 | | |
| 615-145B | Cationic Potato Starch | | | | 0.50 | |
| 615-147A | Cationic Mung Bean Starch | | | | | 0.50 |
| Texapon N 70 NA | Sodium Laureth Sulfate | 12.85 | 12.85 | 12.85 | 12.85 | 12.85 |
| Amphosol CG | Cocamidopropyl Betaine | 8.57 | 8.57 | 8.57 | 8.57 | 8.57 |
| Sodium Chloride USP | Sodium Chloride | 1.00 | 1.00 | 1.00 | 1.00 | 1.00 |
| Fragrance | Fragrance | 0.60 | 0.60 | 0.60 | 0.60 | 0.60 |
| Edeta BD | Disodium EDTA | 0.10 | 0.10 | 0.10 | 0.10 | 0.10 |
| Preservatives, pH Adjuster | | 1.00 | 1.00 | 1.00 | 1.00 | 1.00 |
| | | 100 | 100 | 100 | 100 | 100 |

### Rinse Off Conditioner

**Table 7**

| **Ingredient** | **Common Name** | % | % |
|---|---|---|---|
| DI Water | Water | 87.23 | 87.73 |
| 615-149A | Cationic Potato Starch | 0.50 | |
| 615-147A | Cationic Mung Bean Starch | | 0.50 |
| Edeta BD | Disodium EDTA | 0.02 | 0.02 |
| Lexamine S-13 | Stearamidopropyl Dimethylamine | 2.00 | 2.00 |
| Lanette 16 | Cetyl Alcohol | 2.00 | 2.00 |
| Lanette 18 | Stearyl Alcohol | 4.00 | 4.00 |
| Xiameter PMX-200 Silicone | Dimethicone | 0.75 | 0.75 |
| Xiameter PMX-0345 | Cyclopentasiloxane,Cyclohexasiloxane | 2.00 | 2.00 |
| Fragrance | Fragrance | 0.50 | 0.50 |
| Preservatives, pH Adjuster | | 1.00 | 1.00 |
| | TOTAL | 100 | 100 |

### Leave In Moisturizer

**Table 8**

| **Ingredient** | **Common Name** | **%** | **%** |
|---|---|---|---|
| DI Water | WATER | 95.35 | 95.35 |
| 615-149A | Cationic Potato Starch | 0.35 | |
| 615-147A | Medium Charge Mung Bean Starch | | 0.35 |
| Xiameter OFX-0193 Fluid | PEG-12 Dimethicone | 1.00 | 1.00 |
| Xiameter MEM 0949 | Amodimethicone (and) Cetrimonium Chloride (and)Trideceth-12 | 1.50 | 1.50 |
| Fragrance | Fragrance | 0.40 | 0.40 |
| Brij O 20 | Oleth-20 | 0.40 | 0.40 |
| Preservatives, pH Adjuster | | 1.00 | 1.00 |
| | | 100 | 100 |

### Oil Moisturizer

**Table 9**

| **Ingredient** | **Common Name** | **%** | **%** |
|---|---|---|---|
| DI Water | Water | 85.40 | 85.40 |
| 615-149A | Cationic Potato Starch | 0.50 | |
| 615-147A | Medium Charge Mung Bean Starch | | 0.50 |
| Salcare SC 96 | Polyquaternium-37, Propylene Glycol Dicaprylate/Dicaprate, PPG-1 Trideceth-6 | 2.50 | 2.50 |
| Propylene Glycol USP | Propylene Glycol | 2.00 | 2.00 |
| Rita Coconut Oil 76 | Cocos Nucifera (Coconut) Oil | 4.00 | 4.00 |
| Florasun 90 | Helianthus Annus (Sunflower) Seed Oil | 4.00 | 4.00 |
| Fragrance | Fragrance | 0.60 | 0.60 |
| Preservatives, pH Adjuster | | 1.00 | 1.00 |
| | | 100 | 100 |

### Sulfate Free Shampoo

**Table 10**

| **Ingredient** | **Common Name** | **%** | **%** |
|---|---|---|---|
| DI Water | Water | 56.50 | 56.50 |
| 615-149A | Cationic Potato Starch | 0.80 | |
| 615-147A | Cationic Mung Bean Starch | | 0.80 |
| Amphosol CG | Cocamidopropyl Betaine | 10.00 | 10.00 |
| Lexemul EGDS | Glycol Distearate | 1.00 | 1.00 |
| Edeta BX Powder | Tetrasodium EDTA | 0.10 | 0.10 |
| Fragrance | Fragrance | 0.60 | 0.60 |
| Preservatives, pH Adjuster | | 1.00 | 1.00 |
| Hostapon SG | Sodium Cocoyl Isethionate | 30.00 | 30.00 |
| | | 100 | 100 |

### Body Wash

**Table 11**

| **Ingredient** | **Common Name** | **%** | **%** |
|---|---|---|---|
| DI Water | Water | 66.81 | 66.81 |
| 615-149A | Cationic Potato Starch | 0.35 | |
| 615-147A | Cationic Mung Bean Starch | | 0.35 |
| Glycerin USP | Glycerin | 2.00 | 2.00 |
| Amphosol CG | Cocamidopropyl Betaine | 10.00 | 10.00 |
| Texapon N 70 NA | Sodium Laureth Sulfate | 17.14 | 17.14 |
| Florasun 90 | Helianthus Annus (Sunflower) Seed Oil | 2.00 | 2.00 |
| Edeta BX Powder | Tetrasodium EDTA | 0.10 | 0.10 |
| Fragrance | Fragrance | 0.60 | 0.60 |
| Preservatives, pH Adjuster | | 1.00 | 1.00 |
| | Total | 100 | 100 |

### Styling Lotion

**Table 12**

| **Ingredient** | **Common Name** | **%** | **%** |
|---|---|---|---|
| DI Water | Water | 92.65 | 92.65 |
| 615-149A | Cationic Potato Starch | 0.35 | |
| 615-147A | Cationic Mung Bean Starch | | 0.35 |
| Gafquat 755N | Polyquaternium 11 | 3.00 | 3.00 |
| Solulan 75 Lanolin | PEG-75 Lanolin | 0.50 | 0.50 |
| Xiameter OFX-0193 Fluid | PEG-12 Dimethicone | 1.00 | 1.00 |
| Preservatives, pH Adjuster | | 1.00 | 1.00 |
| Fragrance | Fragrance | **0.50** | 0.50 |
| Brij O 20 | Oleth-20 | 1.00 | 1.00 |
| | | 100 | 100 |

### Combing Cream

**Table 13**

| **Ingredient** | **Common Name** | **%** | **%** |
|---|---|---|---|
| DI Water | Water (Aqua) | 87.25 | 87.25 |
| 615-149A | Cationic Potato Starch | 0.35 | |
| 615-147A | Cationic Mung Bean Starch | | 0.35 |
| Propylene Glycol | Propylene Glycol | 2.00 | 2.00 |
| Styleze W-10 | Polyquaternium - 55 | 2.00 | 2.00 |
| Cetearyl Alcohol | Cetearyl Alcohol | 0.50 | 0.50 |
| Geramin KDMP | Behentrimonium Chloride | 2 | 2 |
| Tinocare Si A1 | Aminopropyl Dimethicone | 0.40 | 0.40 |
| Xiameter PMX-200 Silicone | Dimethicone | 2 | 2 |
| Preservatives, pH Adjuster | | 1.00 | 1.00 |
| Fragrance | Fragrance | 0.50 | 0.50 |
| Salcare SC-96 | Polyquaternium-37, Propylene Glycol Dicaprylate/Dicaprate, PPG-1 Trideceth-6 | 1.50 | 1.50 |
| | | 100 | 100 |

### Skin Moisturizer

**Table 14**

| **Ingredient** | **Common Name** | **%** | **%** |
|---|---|---|---|
| DI Water | Water (Aqua) | 76.00 | 76.00 |
| 615-149A | Cationic Potato Starch | 0.50 | |
| 615-147A | Cationic Mung Bean Starch | | 0.50 |
| Sodium Chloride USP | Sodium Chloride | 1.00 | 1.00 |
| Glycerin USP | Glycerin | 5.00 | 5.00 |
| Xiameter PMX-200 Silicone | Dimethicone | 5.00 | 5.00 |
| Dow Corning ES 5600 | Diglyceryl Tris(Trimethylsiloxy) Silylethyl Dimethicone | 4.00 | 4.00 |
| Finsolv TN | C12 - 15 Alkyl Benzoate | 2.00 | 2.00 |
| Permethyl 101A | Isohexadecane | 2.00 | 2.00 |
| Dow Corning 556 Fluid | Phenyl Trimethicone | 2.00 | 2.00 |
| Preservatives, pH Adjuster | | 1.00 | 1.00 |
| Fragrance | Fragrance | 0.50 | 0.50 |
| Sepigel 305 | Polyacrylamide, c13-14 Isoparaffin, Laureth-7 | 1.00 | 1.00 |
| | | 100 | 100 |

### Sanitizer

**Table 15**

| **Ingredient** | **Common Name** | **1%** | **2%** |
|---|---|---|---|
| DI Water | Water (Aqua) | 32.05 | 32.05 |
| 615-149A | Cationic Potato Starch | 0.40 | |
| 615-147A | Cationic Mung Bean Starch | | 0.40 |
| Denat. Alcohol | Denat. Alcohol | 62.00 | 62.00 |
| Glycerin USP | Glycerin | 5.00 | 5.00 |
| Edeta BX Powder | Tetrasodium EDTA | 0.05 | 0.05 |
| Fragrance | Fragrance | 0.50 | 0.50 |
| | | 100 | 100 |

## Claims

1. A cosmetic or dermatologically acceptable composition comprising:
water;
a second ingredient selected from the group consisting of detergents, non-detersive conditioning agents, aesthetically-modifying agents, protective agents, and mixtures thereof; and
an unthinned cationic starch **characterized by**:
a) an amylopectin/amylose ratio of greater than or equal to 60/40;
b) an apparent cationic molecular weight of greater than or equal to 12 million Daltons; and
c) a positive charge density of from 0.5 meq/g to 2.5 meq/g.

2. The composition of claim 1 wherein the cationic starch is selected from the group consisting of potato, rice, waxy rice and waxy corn starch.

3. The composition of claim 1 which is a shampoo.

4. The composition of claim 1 wherein the non-detersive conditioning agent is selected from the group consisting of oily substances, non-ionic substances, cationic amphiphilic ingredients, cationic non-starch ingredients, solvents, esters, extracts, silicon polymers and polyquarternium ingredients.

5. The composition of claim 1 which is a conditioning shampoo.

6. The composition of claim 1 wherein the cationic starch is present at a concentration of from 0.3% to 3% w/w.

7. The composition of claim 1 wherein the cationic starch is present at a concentration of from 0.5% to 2% w/w.

8. The composition of claim 1 which is free of silicone polymers.

9. The composition of claims 1 or 8 wherein the cationic starch is selected from the group consisting of cationic potato, rice, waxy rice, waxy corn, and mung bean starches.

10. A method of conditioning hair comprising treating hair with a cosmetic or dermatologically acceptable composition comprising:
water;
a second ingredient selected from the group consisting of detergents, non-detersive conditioning agents, aesthetically-modifying agents, protective agents, and mixtures thereof; and
an unthinned cationic starch **characterized by**:
a) an amylopectin/amylose weight ration of greater than 60/40;
b) an apparent cationic molecular weight of greater than or equal to 12 million Daltons; and
c) a positive charge density of from 0.5 meq/g to 2.5 meq/g.

11. The method of claim 10 wherein the non-detersive conditioning agent is selected from the group consisting of oily substances, non-ionic substances, cationic amphiphilic ingredients, cationic non-starch ingredients, solvents, esters, extracts, silicon polymers and polyquarternium ingredients.

12. The method of claim 10 wherein the composition is free of silicone polymers.

13. The method of claims 10 or 13 wherein the cationic starch is selected from the group consisting of cationic potato, rice, waxy rice waxy corn, and mung bean starches.

## Patentansprüche

1. Kosmetische oder dermatologisch verträgliche Zusammensetzung, umfassend:
Wasser;
einen zweiten Inhaltsstoff ausgewählt aus der Gruppe bestehend aus Tensiden, nichtreinigenden, konditionierenden Agentien, ästhetisch-modifizierenden Agentien, schützenden Agentien und Mischungen davon;
und
eine unverdünnte, kationische Stärke, **gekennzeichnet durch**:
a) ein Verhältnis Amylopektin/Amylose größer als oder gleich 60/40;
b) ein scheinbares, kationisches Molekulargewicht größer als oder gleich 12 Millionen Dalton; und
c) eine positive Ladungsdichte von 0,5 meq/g bis 2,5 meq/g.

2. Zusammensetzung nach Anspruch 1, wobei die kationische Stärke ausgewählt ist aus der Gruppe bestehend aus Kartoffel-, Reis-, Wachsreis- und Wachsmaisstärke.

3. Zusammensetzung nach Anspruch 1, die ein Shampoo ist.

4. Zusammensetzung nach Anspruch 1, wobei das nichtreinigende, konditionierende Agens ausgewählt ist aus der Gruppe bestehend aus öligen Substanzen, nichtionischen Substanzen, kationischen, amphiphilen Inhaltsstoffen, kationischen Nicht-Stärke-Inhaltsstoffen, Lösungsmitteln, Estern, Extrakten, Silikonpolymeren und polyquarternären Inhaltsstoffen.

5. Zusammensetzung nach Anspruch 1, die ein Konditionierungsshampoo ist.

6. Zusammensetzung nach Anspruch 1, wobei die kationische Stärke in einer Konzentration von 0,3 Gew.-% bis 3 Gew.-% vorhanden ist.

7. Zusammensetzung nach Anspruch 1, wobei die kationische Stärke in einer Konzentration von 0,5 Gew.-% bis 2 Gew.-% vorhanden ist.

8. Zusammensetzung nach Anspruch 1, die frei von Silikonpolymeren ist.

9. Zusammensetzung nach Ansprüchen 1 oder 8, wobei die kationische Stärke ausgewählt ist aus der Gruppe bestehend aus kationischer Kartoffel-, Reis-, Wachsreis-, Wachsmais- und Mungbohnenstärken.

10. Verfahren der Haarkonditionierung, umfassend das Behandeln des Haares mit einer kosmetischen oder dermatologisch verträglichen Zusammensetzung, umfassend:
Wasser;
einen zweiten Inhaltsstoff ausgewählt aus der Gruppe bestehend aus Tensiden, nichtreinigenden, konditionierenden Agentien, ästhetisch-modifizierenden Agentien, schützenden Agentien und Mischungen davon;
und
eine unverdünnte, kationische Stärke, **gekennzeichnet durch**:
a) ein Gewichtsverhältnis Amylopektin/Amylose größer als 60/40;
b) ein scheinbares, kationisches Molekulargewicht größer als oder gleich 12 Millionen Dalton; und
c) eine positive Ladungsdichte von 0,5 meq/g bis 2,5 meq/g.

11. Verfahren nach Anspruch 10, wobei das nichtreinigende, konditionierende Agens ausgewählt ist aus der Gruppe bestehend aus öligen Substanzen, nichtionischen Substanzen, kationischen, amphiphilen Inhaltsstoffen, kationischen Nicht-Stärke-Inhaltsstoffen, Lösungsmitteln, Estern, Extrakten, Silikonpolymeren und polyquarternären Inhaltsstoffen.

12. Verfahren nach Anspruch 10, wobei die Zusammensetzung frei von Silikonpolymeren ist.

13. Verfahren nach Ansprüchen 10 oder 13, wobei die kationische Stärke ausgewählt ist aus der Gruppe bestehend aus kationischer Kartoffel-, Reis-, Wachsreis-Wachsmais- und Mungbohnenstärken.

## Revendications

1. Composition cosmétique ou dermatologiquement acceptable comprenant :
de l'eau ;
un deuxième ingrédient sélectionné dans le groupe consistant en les détergents, les agents revitalisants non détersifs, les agents de modification esthétique, les agents protecteurs, et les mélanges de ceux-ci ; et
un amidon cationique non dilué **caractérisé par** :
a) un rapport amylopectine/amylose supérieur ou égal à 60/40 ;
b) une masse moléculaire cationique apparente supérieure ou égale à 12 millions de Daltons ; et
c) une densité de charge positive de 0,5 meq/g à 2,5 meq/g.

2. Composition selon la revendication 1, dans laquelle l'amidon cationique est sélectionné dans le groupe consistant en l'amidon de pomme de terre, de riz, de riz cireux et de maïs cireux.

3. Composition selon la revendication 1 qui est un shampooing.

4. Composition selon la revendication 1 dans laquelle l'agent revitalisant non détersif est sélectionné dans le groupe consistant en des substances huileuses, des substances non ioniques, des ingrédients amphiphiles cationiques, des ingrédients non amidons cationiques, des solvants, des esters, des extraits, des polymères de silicone et des ingrédients polyquaternaires.

5. Composition selon la revendication 1 qui est un shampooing revitalisant.

6. Composition selon la revendication 1 dans laquelle l'amidon cationique est présent en une concentration de 0,3 % à 3 % p/p.

7. Composition selon la revendication 1 dans laquelle l'amidon cationique est présent en une concentration de 0,5 % à 2 % p/p.

8. Composition selon la revendication 1 qui est dépourvue de polymères de silicone.

9. Composition selon les revendications 1 ou 8 dans laquelle l'amidon cationique est sélectionné dans le groupe consistant en les amidons cationiques de pomme de terre, de riz, de riz cireux, de maïs cireux et de haricot mungo.

10. Procédé de revitalisation des cheveux comprenant le traitement des cheveux avec une composition cosmétique ou dermatologiquement acceptable comprenant :
de l'eau ;
un deuxième ingrédient sélectionné dans le groupe consistant en les détergents, les agents revitalisants non détersifs, les agents de modification esthétique, les agents protecteurs, et les mélanges de ceux-ci ; et
un amidon cationique non dilué **caractérisé par** :
a) un rapport pondéral amylopectine/amylose supérieur à 60/40 ;
b) une masse moléculaire cationique apparente supérieure ou égale à 12 millions de Daltons ; et
c) une densité de charge positive de 0,5 meq/g à 2,5 meq/g.

11. Procédé selon la revendication 10 dans lequel l'agent revitalisant non détersif est sélectionné dans le groupe consistant en des substances huileuses, des substances non ioniques, des ingrédients amphiphiles cationiques, des ingrédients non amidons cationiques, des solvants, des esters, des extraits, des polymères de silicone et des ingrédients polyquaternaires.

12. Procédé selon la revendication 10 dans lequel la composition est dépourvue de polymères de silicone.

13. Procédé selon les revendications 10 ou 13 dans lequel l'amidon cationique est sélectionné dans le groupe consistant en les amidons cationiques de pomme de terre, de riz, de riz cireux, de maïs cireux et de haricot mungo.
